# EUROPEAN PATENT APPLICATION

(11) **EP 1 791 092 A2**
(43) Date of publication of application: **30.05.2007**
(21) Application number: 06124545.2
(22) Date of filing: 22.11.2006
(51) Int. Cl.: G06T 17/40

(54) **System and method for radiology decision support using picture-in-picture**

(30) Priority: 23.11.2005 US 286665
(71) Applicant: GENERAL ELECTRIC COMPANY, Schenectady, NY 12345 (US)
(72) Inventor: Mahesh, Prakash, Hoffman Estates, IL 60192 (US); Stoval III, William Murray, Mount Prospect, IL 60056 (US); Kariathungal, Murali Kumaran, Hoffman Estates, IL 60194 (US)
(74) Representative: Bedford, Grant Richard

(57) **Abstract**

Certain embodiments of the present invention provide for a system for viewing medical images including a set of image slices, a selected image (110), an overview image (120, 200), and a user interface component (100). The selected image (110) is selected from the set of image slices. The overview image (120, 200) includes a representation (210) of the set of image slices. The user interface component (100) is capable of simultaneously displaying the selected image (110) and the overview image (120, 200).

## Description

The present invention generally relates to viewing medical images. In particular, the present invention relates to a system and method for radiology decision support using picture-in-picture.

Healthcare environments, such as hospitals or clinics, include clinical information systems, such as hospital information systems (HIS) and radiology information systems (RIS), and storage systems, such as picture archiving and communication systems (PACS). Information stored may include patient medical histories, imaging data, test results, diagnosis information, management information, and/or scheduling information, for example. The information may be centrally stored or divided at a plurality of locations. Healthcare practitioners may desire to access patient information or other information at various points in a healthcare workflow. For example, during surgery, medical personnel may access patient information, such as images of a patient's anatomy, that are stored in a medical information system. Alternatively, medical personnel may enter new information, such as history, diagnostic, or treatment information, into a medical information system during an ongoing medical procedure.

PACS connect to medical diagnostic imaging devices and employ an acquisition gateway (between the acquisition device and the PACS), storage and archiving units, display workstations, databases, and sophisticated data processors. These components are integrated together by a communication network and data management system. A PACS has, in general, the overall goals of streamlining health-care operations, facilitating distributed remote examination and diagnosis, and improving patient care.

A typical application of a PACS system is to provide one or more medical images for examination by a medical professional. For example, a PACS system can provide a series of x-ray images to a display workstation where the images are displayed for a radiologist to perform a diagnostic examination. Based on the presentation of these images, the radiologist can provide a diagnosis. For example, the radiologist can diagnose a tumor or lesion in x-ray images of a patient's lungs.

A reading, such as a radiology or cardiology procedure reading, is a process of a healthcare practitioner, such as a radiologist or a cardiologist, viewing digital images of a patient. The practitioner performs a diagnosis based on a content of the diagnostic images and reports on results electronically (e.g., using dictation or otherwise) or on paper. The practitioner, such as a radiologist or cardiologist, typically uses other tools to perform diagnosis. Some examples of other tools are prior and related prior (historical) exams and their results, laboratory exams (such as blood work), allergies, pathology results, medication, alerts, document images, and other tools.

A clinical or healthcare environment is a crowded, demanding environment that would benefit from organization and improved ease of use of imaging systems, data storage systems, and other equipment used in the healthcare environment. A healthcare environment, such as a hospital or clinic, encompasses a large array of professionals, patients, and equipment. Personnel in a healthcare facility must manage a plurality of patients, systems, and tasks to provide quality service to patients. Healthcare personnel may encounter many difficulties or obstacles in their workflow.

A variety of distractions in a clinical environment may frequently interrupt medical personnel or interfere with their job performance. Furthermore, workspaces, such as a radiology workspace, may become cluttered with a variety of monitors, data input devices, data storage devices, and communication device, for example. Cluttered workspaces may result in inefficient workflow and service to clients, which may impact a patient's health and safety or result in liability for a healthcare facility. Data entry and access is also complicated in a typical healthcare facility.

In radiology, every image taken during an exam may require a radiologist's examination. The radiologist views each image, marks down several findings and notes the findings in a report. A radiologist associates findings with an image location and provides appropriate descriptions of findings based on the image coordinates and anatomical location.

Current imaging systems may generate a large number of images for a radiologist to examine. For example, while a computed tomography (CT) scanner may generate a set or stack of only a few image slices, those slices may be used to generate one or more three-dimensional (3D) volumetric data sets from which multiple series or sets of thousands of image slices may be generated. 3D-based diagnosis is still a maturing field, and more than 95% of reading is based on two-dimensional viewings of these large image sets. Typically, a radiologist relies on a combination of automatic and manual cine viewing of the images. In a cine viewing, the images are presented to the viewer in sequence, similar to watching a movie progressing through the images in the series.

With increasing volumes of examinations and images, a reduction of radiologists and mounting pressures on improved productivity, radiologists and other healthcare personnel are in need of image processing or display workflow enhancements that allow them to verify they have viewed all the images available to them. Current systems do not provide a way for a radiologist to be sure he has looked at all the images. With such large sets of images to review, a radiologist may not even realize some of the images were not seen. If some images are not viewed, problems may be missed. For example, if a radiologist stops reviewing images after a single anomaly has been found, other anomalies and/or abnormalities may go undetected, and thus untreated.

Another problem caused by the large number of images is that a radiologist may not spend sufficient time reviewing each image. For example, in manual cine mode, a radiologist may skip past images simply due to the sheer volume to get through. If insufficient time is spent reviewing an image, an important feature may not be noticed.

Computer-aided diagnosis (CAD) of image data may be utilized by practitioners to aid in reading medical images. CAD software can identify features and potential abnormalities, and/or anomalies in medical images to bring to the attention of the practitioner. The CAD software then generates markers indicating these features, abnormalities, and/or anomalies on the image. For example, the CAD software may overlay arrows pointing out a potential abnormality in the image. In addition, CAD software can generate a report of the identified features, abnormalities, and/or anomalies. The practitioner may then review the marked images and/or reports prior to making a final diagnosis. Current systems, however, do not provide a way for a radiologist to verify he has viewed all of the marked images.

Therefore, there is a need for a system and method for radiology decision support using picture-in-picture.

Certain embodiments of the present invention provide for a system for viewing medical images including a set of image slices, a selected image, an overview image, and a user interface component. The selected image is selected from the set of image slices. The overview image includes a representation of the set of image slices. The user interface component is capable of simultaneously displaying the selected image and the overview image.

Certain embodiments include a processing component. The processing component is capable of selecting the selected image from the set of image slices. The processing component is capable of generating the overview image. In an embodiment, the overview image is overlaid at least in part on the selected image. In an embodiment, the overview image is adjacent to the selected image. In an embodiment, the overview image includes an indicator of the position of the selected image in the set of image slices. In an embodiment, the overview image includes a representation of the view state of one or more image slices in the set of image slices. In an embodiment, the representation of the view state is color coded. In an embodiment, the view state for an image slice in the set of image slices is based at least in part on the amount of time the image slice is displayed as the selected image. In an embodiment, the overview image includes an overview marker. The overview marker is based at least in part on an image marker in an image slice in the set of image slices. In an embodiment, the image marker is determined at least in part by a computer-aided diagnosis (CAD) system. In an embodiment, the overview image includes a representation of the view state of the image marker. In an embodiment, the representation of the view state of the image marker is color coded.

Certain embodiments of the present invention provide for a method for improving workflow in medical imaging including displaying a selected image and displaying an overview image. The selected image is selected from a set of image slices. The overview image is based at least in part on the set of image slices. The selected image and the overview image are displayed simultaneously.

Certain embodiments include selecting the selected image from the overview image. Certain embodiments include displaying on the overview image a representation of the view state of one or more image slices in the set of image slices. Certain embodiments include displaying on the overview image a representation of the view state of a marker in an image slice in the set of image slices. Certain embodiments include notifying a user. In an embodiment, the user is notified when not all of the image slices in the set of image slices have been displayed as the selected image. In an embodiment, the user is notified when not all of the image slices in the set of image slices have been displayed as the selected image for a pre-determined amount of time. In an embodiment, one or more image slices in the set of image slices include one or more image markers. In an embodiment, the user is notified when not all of the image slices including the image markers have been displayed as the selected image.

Certain embodiments of the present invention provide for a computer-readable medium including a set of instructions for execution on a computer, the set of instructions including a selection display routine, an overview display routine, and a processing routine. The selection display routine is configured to display a selected image. The selected image is based at least in part on an image selected from a set of image slices. The overview display routine is configured to display an overview image simultaneously with the selected image. The overview image includes a representation of the set of image slices. The processing routine is configured to overlay a representation of the view state of one or more of the images in the set of image slices on the overview image.

Various aspects and embodiments of the present invention will now be described in connection with the accompanying drawings, in which:
Figure 1 illustrates an interface for a system for viewing medical images used in accordance with an embodiment of the present invention.
Figure 2 illustrates an overview image used in accordance with an embodiment of the present invention.
Figure 3 illustrates a flow diagram for a method for improving workflow in viewing medical images.

The foregoing summary, as well as the following detailed description of certain embodiments of the present invention, will be better understood when read in conjunction with the appended drawings. For the purpose of illustrating the invention, certain embodiments are shown in the drawings. It should be understood, however, that the present invention is not limited to the arrangements and instrumentality shown in the attached drawings.

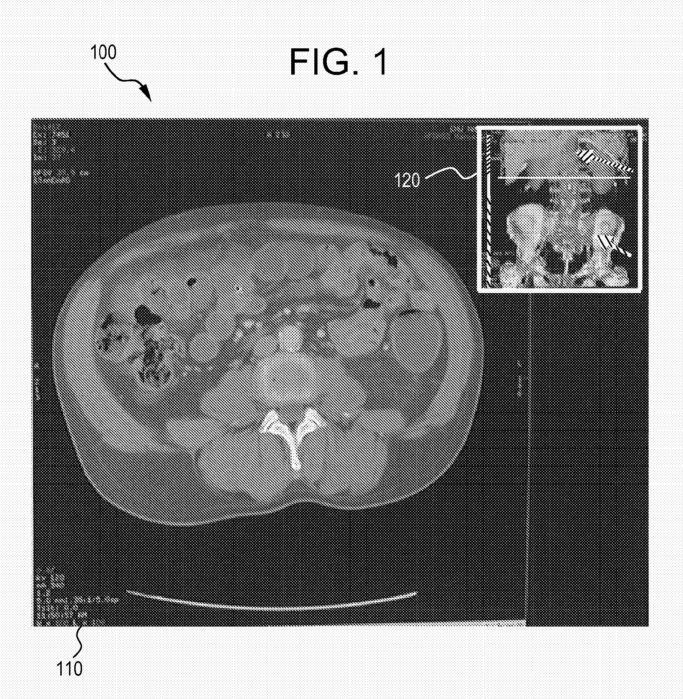

Figure 1 illustrates an interface 100 for a system for viewing medical images used in accordance with an embodiment of the present invention. The interface 100 includes a selected image 110 and an overview image 120.

In operation, the selected image 110 and the overview image 120 are displayed simultaneously. The selected image 110 and the overview image 120 may be displayed by a user interface component (not shown), for example.

The selected image 110 is selected from a set of image slices (not shown). The set of image slices may also be referred to a stack of image slices. The set of image slices may be, for example, slices from an acquisition modality, generated from a 3D volumetric data set, or stored in a PACS. For example, the set of image slices may be a series of image slices generated by a CT scanner. As another example, the set of image slices may be generated from a 3D volumetric data set based on a prescription by a radiologist.

The overview image 120 includes a representation of the set of image slices. The representation may be a 3D representation, for example. As another example, the representation may be an image slice used to generate a 3D volumetric data set from which the set of image slices was generated. As another example, the representation may be a scout image.

In an embodiment, the overview image 120 is displayed and/or overlaid on the selected image 110. For example, the overview image 120 may be displayed inset on the selected image 110, as depicted in Figure 1. In an embodiment, overview image 120 may be displayed adjacent to selected image 110. For example, the selected image 110 may be displayed on the left-hand side of a display and the selected image 120 may be displayed on the right-hand portion of the display. In certain embodiments, the selected image 110 is displayed larger than the overview image 120. In certain embodiments, the selected image 110 is displayed smaller than or equal in size to the overview image 120.

In an embodiment, the selected image 110 and the overview image 120 are displayed on the same display. For example, the selected image 110 and the overview image 120 may be displayed on a computer monitor, tablet computer, or other display device. In an embodiment, the selected image 110 and the overview image 120 are displayed on different displays. For example, the selected image 110 may be displayed on one monitor connected to a computer and the overview image may be displayed on a second monitor connected to the computer.

In an embodiment, a user may select the selected image 110 to display with the overview image 120. For example, a user may select a location in the overview image 120 and the image slice including or nearest to the location the user selected will then be displayed as the selected image 110. The user may select a location by clicking with a mouse or manipulating a cursor, for example.

In an embodiment, the selected image 110 may be selected from the set of image slices automatically. For example, in a cine mode, the selected image 110 will be selected from the set sequentially, starting at one end and progressing through the set, with each slice displayed as the selected image 110 for a pre-determined amount of time. For example, each selected image 110 may be shown for 0.5 seconds.

In certain embodiments, the interface 100 may be in communication with a processing component (not shown). In an embodiment, a processing component (not shown) generates the overview image 120 based at least in part on the set of image slices. In an embodiment, a processing component selects the selected image 110 from the set of image slices. The processing component may select the selected image 110 based at least in part on input from a user, for example. The processing component may select the selected image 110 automatically, for example. For example, the processing component may select the selected image 110 by sequentially selecting images from the set of image slices as part of a cine view mode.

The components, elements, and/or functionality of interface 100 and the corresponding components and/or system may be implemented alone or in combination in various forms in hardware, firmware, and/or as a set of instructions in software, for example. Certain embodiments may be provided as a set of instructions residing on a computer-readable medium, such as a memory or hard disk, for execution on a general purpose computer or other processing device, such as, for example, a PACS workstation or one or more dedicated processors.

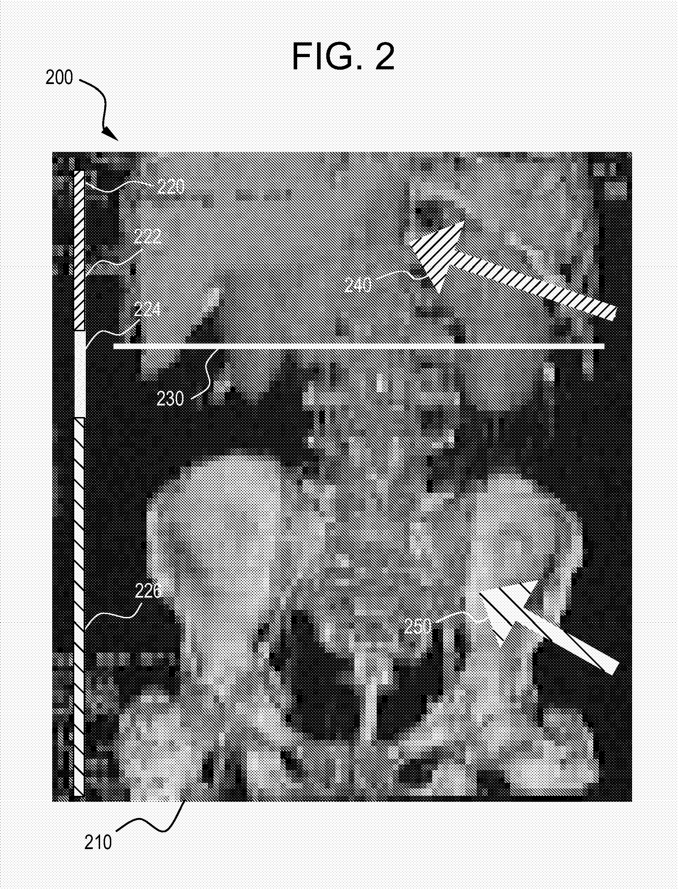

Figure 2 illustrates an overview image 200 used in accordance with an embodiment of the present invention. The overview image 200 includes a representation 210 of a set of image slices, a representation 220 of a view state of images in the set of image slices, an indicator 230 of a position of a selected image slice, and overview markers 240, 250.

The overview image 200 may be similar to the overview image 120, described above, for example. In certain embodiments, overview image 200 may not include the representation 220 of the view state of the set of image slices, the indicator 230 of the position of a selected image slice, and/or overview markers 240, 250.

In operation, the overview image 200 includes the representation 210 of a set of image slices. The representation 210 may be a 3D representation, for example. As another example, the representation 210 may be an image slice used to generate the 3D volumetric data set from which the set of image slices was generated. As another example, the representation 210 may be a scout image. The set of image slices may be, for example, slices from an acquisition modality or generated from a 3D volumetric data set. For example, the set of image slices may be a series of image slices generated by a CT scanner. As another example, the set of image slices may be generated from a 3D volumetric data set based on a prescription by a radiologist.

The representation 220 of the view state of the set of image slices may be overlaid on the representation 210 of the set of image slices. The representation 220 of the view state of the set of image slices refers to a representation of the view state of one or more image slices in the set of image slices. The view state of an image slice refers to, for example, whether a particular image slice in the set of image slices has been displayed as a selected image. For example, the view state of an image slice may be a binary value, indicated whether or not the image slice has been displayed as a selected image. As another example, the view state of an image slice may be one of an enumerated set of states. The view state of an image slice may be represented in the representation 220 of the view state of the set of image slices by a color, symbol, or other indicator, for example.

In an embodiment, the view state of an image slice may also reflect the amount of time the image slice was displayed. For example, if an image slice has been displayed as the selected image for less than 0.5 seconds, the representation of the view state for that image slice may be different than a representation for an image slice that has either not been displayed as a selected image or that has been displayed for longer than a minimum amount of time. That is, an image slice may have view states indicating 'not displayed,' 'displayed for less than 0.5 seconds,' and 'displayed for 0.5 seconds or greater.' Such a view state indication may assist a user in verifying an image slice was not only displayed as a selected image, but also that it was examined by the user for a sufficient amount of time, for example. Additional view states may be represented. For example, view states may be represented for image slices that have not been displayed, those that have been displayed for less than a second, those that have been displayed for one to three seconds, and those that have been displayed for more than three seconds.

In an embodiment, the representation 220 of the view state of the set of image slices is color coded. For example, image slices that have displayed for a certain amount of time may be colored green in the representation 220 of the view state for the corresponding image slices. As another example, image slices that have been displayed, but not for a certain amount of time, may be colored yellow in the representation 220 of the view state for the corresponding image slices. As another example, image slices that have not been displayed may be colored red in the representation 220 of the view state for the corresponding image slices. As illustrated in Figure 2, for example, the subset of the image slices that have been displayed for at least some minimum amount of time are indicated with green 222 on the representation 220 of the view state of the set of image slices. The subset of the image slices that have been displayed, but not for some minimum amount of time, are indicated with yellow 224 on the representation 220 of the view state of the image slices. The subset of the image slices that have not been displayed are indicated with red 226 on the representation 220 of the view state of the image slices.

The position of the currently displayed selected image may be displayed on the overview image 200 with an indicator 230 of the position of the image slice in the set of image slices. The indicator 230 may illustrate the location of the image slice corresponding to the selected image with respect to the representation 210 of the set of image slices. For example, the indicator 230 may be a line, arrow, or other marker indicating the position of the plane of the image slice corresponding to the currently displayed selected image.

In an embodiment, a user may change the selected image by dragging the indicator 230. That is, the selected image will be changed to the image slice at the new position of the indicator 230. In an embodiment, a user may select a location on the representation 210 of the set of image slices and the image slice including or nearest to the location the user selected will then be displayed as the selected image. The user may select a location by clicking with a mouse or manipulating a cursor, for example. The indicator 230 may then update to display the location of the new selected image in the set of image slices.

The overview image 200 may include one or more overview markers such as overview markers 240, 250. An image slice in the set of image slices may include one or more image markers. An image marker may indicate a feature, anomaly, and/or abnormality of possible interest to the viewer of the image slice, for example. An image marker may be generated by a CAD system, for example. An overview marker, such as overview markers 240, 250, may be overlaid and/or presented on the overview image 200 to indicate the location of an corresponding image marker on an image slice. An overview marker may be a symbol such as, for example, an arrow, circle, cross, rectangle, or other shape to indicate the location of the corresponding image marker.

In an embodiment, one overview marker is included in the overview image 200 for each corresponding image marker on an image slice in the set of image slices. In an embodiment, an overview marker corresponds to several proximate image markers.

In addition to indicating the location of an image marker, an overview marker may also indicate the view state of the corresponding image marker. This indication of view state by the overview marker may be similar to the representation 220 of view state of image slices in the set of image slices, described above, for example. For example, the view state of an image marker may indicate that an image slice containing that image marker has not been displayed.

In an embodiment, the overview marker is color coded to indicate the view state of the corresponding image marker. This color coding may be similar to the color coding in the representation 220 of the view state of image slices in the set of image slices, described above, for example. For example, the overview marker may be green (e.g., overview marker 240) when the image slice including the corresponding image marker has been displayed as the selected image. As another example, the overview marker may be red (e.g., overview marker 240) when the image slice including the corresponding image marker has not been displayed as the selected image. In an embodiment, the color coding convention for the view state of the image marker corresponds to the color coding convention for the view state of the image slices in the set of image slices, described above.

In certain embodiments, a user may receive a notification when trying to exit the viewing application or attempting to view a different set of image slices, for example. In an embodiment, a user receives a notification when not all of the image slices have been displayed. That is, a notification is made when the view state of one or more image slices in the set of image slices indicates the image slice has not been displayed as a selected image. In an embodiment, a user receives a notification when not all of the image slices have been displayed for a certain amount of time. That is, a notification is made when the view state of one or more image slices in the set of image slices indicates the image slice has not been displayed as a selected image for a certain period of time.

In an embodiment, a user receives a notification when not all of the image markers have been displayed. That is, a notification is made when the view state of one or more image markers indicates the image slice including the image marker has not been displayed as a selected image. In an embodiment, a user receives a notification when not all of the image markers have been displayed for a certain amount of time. That is, a notification is made when the view state of one or more image markers indicates the image slice including the image marker has not been displayed as a selected image for a certain period of time.

The components, elements, and/or functionality of overview image 200 and the corresponding components and/or system may be implemented alone or in combination in various forms in hardware, firmware, and/or as a set of instructions in software, for example. Certain embodiments may be provided as a set of instructions residing on a computer-readable medium, such as a memory or hard disk, for execution on a general purpose computer or other processing device, such as, for example, a PACS workstation or one or more dedicated processors.

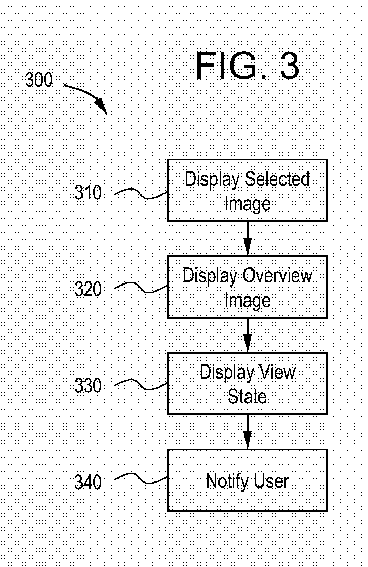

Figure 3 illustrates a flow diagram for a method 300 for improving workflow in viewing medical images. The method 300 includes the following steps, which will be described below in more detail. At step 310, a selected image is displayed. At step 320, an overview image is displayed. At step 330, view state is displayed. At step 340, a user is notified. The method 300 is described with reference to elements of systems described above, but it should be understood that other implementations are possible.

At step 310, a selected image is displayed. The selected image is selected from a set of image slices. The selected image may be similar to the selected image 110, described above, for example. In an embodiment, the selected image is displayed by a user interface component. The selected image may be displayed on a computer monitor, tablet computer, or other display device, for example. In an embodiment, the selected image is selected by a processing component.

At step 320, an overview image is displayed. The overview image is a representation of a set of image slices. The overview image may be similar to overview image 120, described above, for example. The overview image may be similar to overview image 200, described above, for example. In an embodiment, the overview image is displayed by a user interface component. The overview image may be displayed on a computer monitor, tablet computer, or other display device, for example. In an embodiment, the overview image is generated by a processing component.

The overview image is displayed simultaneously with the selected image displayed at step 310, described above. In an embodiment, the overview image is displayed and/or overlaid on the selected image. For example, the overview image may be displayed inset on the selected image, as depicted in Figure 1. In an embodiment, overview image may be displayed adjacent to selected image. For example, the selected image may be displayed on the left-hand side of a display and the selected image may be displayed on the right-hand portion of the display.

At step 330, view state is displayed. The view state of one or more image slices in a set of image slices may be displayed on the overview image. The overview image may be similar to the overview images 120, 200, discussed above, for example.

The view state of an image slice may be similar to the view state of an image slice, as discussed above, for example. The displayed view state may be similar to the representation 220 of the view state of image slices in the set of image slices, discussed above, for example. In an embodiment, the displayed view state is similar to the view state for image markers, discussed above.

At step 340, a user is notified. The user may be given a notification similar to notification discussed above, for example. The user may be notified when the user attempts to cease displaying selected images from the set of image slices, for example, when the user has not displayed all of the images in the set of image slices. That is, the user may be notified when the view state for an image slice in the set of image slices indicates the image slice has not been displayed as a selected image and the user attempts to exit the viewing system or switch to another set of image slices, for example. As another example, the user may be notified when attempting to cease displaying the set of image slices when the user has not displayed all of the image slices in the set of image slices for a certain amount of time.

One or more of the steps of the method 300 may be implemented alone or in combination in hardware, firmware, and/or as a set of instructions in software, for example. Certain embodiments may be provided as a set of instructions residing on a computer-readable medium, such as a memory or hard disk, for execution on a general purpose computer or other processing device, such as, for example, a PACS workstation or image viewer.

Certain embodiments of the present invention may omit one or more of these steps and/or perform the steps in a different order than the order listed. For example, some steps may not be performed in certain embodiments of the present invention. As a further example, certain steps may be performed in a different temporal order, including simultaneously, than listed above.

Thus, certain embodiments provide a system and method for radiology decision support using picture-in-picture. Certain embodiments allow the view state of images and/or image markers in a set of image slices to be presented to a user. Certain embodiments allow a user to be notified when one or more image slices and/or image markers have not be viewed or may not have been adequately viewed.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. Therefore, it is intended that the invention not be limited to the particular embodiment disclosed, but that the invention will include all embodiments falling within the scope of the appended claims.

### Parts List

| | |
|---|---|
| **Interface for System for Viewing Medical Images** | **100** |
| **Selected Image** | **110** |
| **Overview Image** | **120** |
| **Overview Image** | **200** |
| **Representation of Set of Image Slices** | **210** |
| **Representation of View State of Set of Image Slices** | **220** |
| **Green Representation of View State** | **222** |
| **Yellow Representation of View State** | **224** |
| **Red Representation of View State** | **226** |
| **Indicator of Position of Selected Image Slice** | **230** |
| **Overview Marker** | **240** |
| **Overview Marker** | **250** |
| **Flow Diagram** | **Fig. 3** |

## Claims

1. A system for viewing medical images, the system including:
a set of image slices;
a selected image (110), wherein the selected image (110) is selected from the set of image slices;
an overview image (120, 200), wherein the overview image (120, 200) includes a representation (210) of the set of image slices;
a user interface component (100), wherein the user interface component (100) is capable of simultaneously displaying the selected image (110) and the overview image (120, 200).

2. The system of claim 1, wherein the overview image (120, 200) includes an indicator (230) of the position of the selected image (110) in the set of image slices.

3. The system of claim 1 or claim 2, wherein the overview image (120, 200) includes a representation (220) of the view state of one or more image slices in the set of image slices.

4. The system of claim 3, wherein the view state for an image slice in the set of image slices is based at least in part on the amount of time the image slice is displayed as the selected image (110).

5. The system of any preceding claim, wherein the overview image (120, 200) includes an overview marker (240, 250), wherein the overview marker (240, 250) is based at least in part on an image marker in an image slice in the set of image slices, wherein the image marker is determined at least in part by a computer-aided diagnosis (CAD) system.

6. The system of claim 5, wherein the overview image (120, 200) includes a representation of the view state of the image marker.

7. A method for improving workflow in medical imaging, the method including:
displaying a selected image (110), wherein the selected image (110) is selected from a set of image slices; and
displaying an overview image (120, 200), wherein the overview image (120, 200) is based at least in part on the set of image slices, wherein the selected image (110) and the overview image (120, 200) are displayed simultaneously.

8. The method of claim 7, furthering including selecting an image slice from the overview image (120, 200), wherein the selected image (110) changes based at least in part on the selected image slice.

9. The method of claim 7 or claim 8, further including displaying on the overview image (120, 200) a representation (2120) of the view state of one or more image slices in the set of image slices.

10. The method of any one of claims 7 to 9, further including notifying a user, wherein the user is notified when not all of the image slices in the set of image slices have been displayed as the selected image (110) for a pre-determined amount of time.
